(19) Europäisches Patentamt
European Patent Office
Office européen des brevets

(11) Publication number: **0 325 288 B1**

(12) **EUROPEAN PATENT SPECIFICATION**

(45) Date of publication of patent specification: **01.09.93**

(51) Int. Cl.[5]: **C07D 209/48**, C07D 209/66, D06L 3/02, C11D 3/395

(21) Application number: **89101002.7**

(22) Date of filing: **20.01.89**

(54) **Imido-aromatic percarboxylic acids.**

(30) Priority: **20.01.88 IT 1913288**

(43) Date of publication of application:
**26.07.89 Bulletin 89/30**

(45) Publication of the grant of the patent:
**01.09.93 Bulletin 93/35**

(84) Designated Contracting States:
**AT BE CH DE ES FR GB LI NL SE**

(56) References cited:
**EP-A- 0 170 386**

**JOURNAL OF THE CHEMICAL SOCIETY, 1962, Part III, pages 2837-4264, The Chemical Society, London, GB; K. BALENOVIC et al.: "Preparation of some peroxy-acids derived from optically active amino-acids"**

(73) Proprietor: **AUSIMONT S.p.A.**
**Foro Buonaparte, 31**
**I-20121 Milano(IT)**

(72) Inventor: **Venturello, Carlo, Dr.**
**Via XXIII Marzo 135**
**I-28100 Novara(IT)**
Inventor: **Cavallotti, Claudio**
**Via Lorenteggio 3**
**I-20146 Milano(IT)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to (poly)percarboxylic acids, which may be referred to as imido-aromatic (poly)percarboxylic acids, to a process for the preparation thereof and to their use in detergent formulations.

In particular, the present invention relates to imido-aromatic (poly)percarboxylic acids having general formula (I):

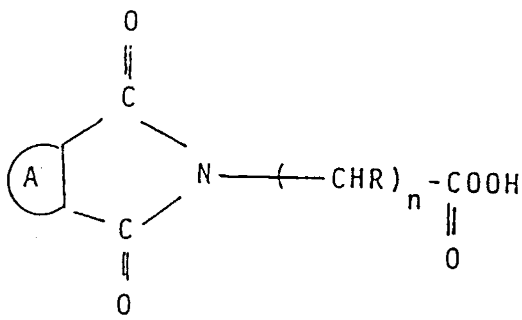

(I)

wherein: A represents an optionally substituted benzene or naphthalene ring as defined below, the group or groups R, which may be the same or different from one another, represent hydrogen, an optionally substituted alkyl group as defined below, COOH or COOOH and n is an integer of from 1 to 5, preferably from 1 to 3.

The (linear or branched) alkyl groups R have 1 to 5 carbon atoms. Examples of $C_{1-5}$ alkyl groups are methyl, ethyl, n- and i-propyl, n-, i-, sek.- and tert.-butyl and pentyl. Said groups may optionally be substituted with one or more (preferably 1 to 3) radicals selected from $C_{1-5}$ alkoxy (e.g. methoxy and ethoxy), OH, COOH or COOOH, COOR' (R' = $C_{1-5}$ alkyl), $NO_2$ and halogen (e.g. F, Cl and Br). Examples of such groups are $-CH_2OCH_3$, $-CH_2OC_2H_5$, $CH_2OH$, $-CH_2COOH$, $-CH_2COOOH$, $-CH_2CH_2COOH$, $-CH_2CH_2COOOH$, $CH_2Cl$, $-CH_2F$, $CF_3$ and $-CH_2COOC_2H_5$.

Likewise, the group A may carry one or more (up to 4 and preferably 1 or 2) substituents. Said substituents are selected from the ones recited above as substituents for the alkyl groups R and, additionally, from $C_{1-5}$ alkyl groups. Particularly preferred substituents are COOH and COOOH radicals. Preferred (optional) substituents for the $C_{1-5}$ alkyl groups R' are the same as those already mentioned above in connection with the groups R.

A particularly preferred meaning of R is hydrogen.

With the exception of L-$\alpha$-phthalimidoperoxypropionic acid, L-$\beta$-phthalimidoperoxybutyric acid and $\alpha$-methyl hydrogen N-phthaloyl-L-peroxyglutamate (already disclosed in J. Chem. Soc., 1962, part 3, p. 3821-2) the imido-aromatic (poly)percarboxylic acids of general formula (I) are novel compounds, and constitute a new class of products that is highly interesting from an industrial point of view.

In fact, they may find general use, similar to that already known for peroxyacids, in the field of plastics, as polymerization initiators and, in particular, as oxidizing agents in epoxidation and hydroxylation, and in many other oxidation processes in the field of fine chemicals.

Particularly, the imido-aromatic (poly)percarboxylic acids having the above formula (I) are particularly suitable as effective bleaching agents in detergent formulations.

In the past, organic peracids have encountered increasing interest in the industrial field, among others due to their excellent properties as bleaching agents in formulations for medium-low temperature washing, and the resulting saving of energy.

Therefore, there is a large number of publications concerned with organic peracid compounds endowed with the required properties of sufficient bleaching activity, and, in particular, thermal stability, the latter characteristics being essential for an industrial application and a widespread use of such compounds.

Therefore many organic straight-chain or cyclic mono- or dipercarboxylic acids are known and used, e.g. in detergent compositions.

Examples of already described percarboxylic acids are diperdodecanedioic acid, monoperphthalic acid, diperazelaic acid, substituted diperglutaric and adipic acids, etc.

Therefore, one object of the present invention is to provide, as per se novel compounds, the imido-aromatic (poly)percarboxylic acids having the above formula (I) (with the exception of the three compounds already known and mentioned above).

Another object is to provide a simple, inexpensive process for the preparation of the above percarboxylic acids of formula (I).

A further object is the use of imido-aromatic percarboxylic acids of formula (I) as bleaching agents in detergent formulations, particularly those intended for low-medium temperature use.

These, and still other objects which will become even clearer for those skilled in the art from the following description, are achieved, according to the present invention, by the imido-aromatic (poly)-percarboxylic acids of the above formula (I), and by the corresponding preparation process, which is characterized in that a substrate selected from imido-aromatic (poly)carboxylic acids and anhydrides thereof having a structure corresponding to the desired percarboxylic acid of formula (I), is reacted with concentrated $H_2O_2$, in a reaction medium selected from concentrated $H_2SO_4$ and $CH_3SO_3H$ or in an alkaline medium, and in that the percarboxylic acid (I) is then separated from the reaction mixture by conventional and known techniques.

In this manner the percarboxylic acids of formula (I) may be obtained, generally as stable solids.

Particularly, preferred compounds of formula (I) according to the present invention are phthalimido-peracetic acid, 3-phthalimido-perpropionic acid 4-phthalimido-perbutyric acid, 2-phthalimido-di-perglutaric acid, 2-phthalimido-dipersuccinic acid, 3-phthalimido-perbutyric acid, 2-phthalimido-perpropionic acid, 3-phthalimido-di-peradipic acid, naphthalimido-peracetic acid, 2-phthalimido-mono-persuccinic acid and 4-(4-percarboxy)-phthalimido-perbutyric acid.

As stated above said peracids may be obtained according to substantially conventional methods, by the reaction of a substrate selected from the imido-aromatic (poly)carboxylic acids of a structure corresponding to the desired peracids of formula (I) with $H_2O_2$ in sulphuric or methanesulphonic acid and by subsequent separation and so forth, according to known techniques, or by working in an alkaline medium, according to known methods, starting from the corresponding anhydrides.

When at least one -(CHR)- residue present in the formula of the starting substrate comprises a carboxylic group it is possible to prepare the corresponding peracid of formula (I) by using the corresponding anhydride.

In this case, depending on the reaction conditions (acidic or alkaline medium and so forth) di- or mono-peracids, i.e. compounds containing two percarboxylic acid groups or one percarboxylic acid group and one carboxylic acid group may selectively be obtained.

According to a preferred procedure, the percarboxylation reaction of the acid or poly-acid used as the starting substrate, is carried out by gradually adding $H_2O_2$, having a concentration of from about 70% to about 90% by weight, to a solution of the acid in concentrated $H_2SO_4$, or in $CH_3SO_3H$, maintaining the reaction temperature throughout the reaction within the range of from about 15° to about 50°C, depending on the reactivity of the substrate.

The amount of $H_2SO_4$ or $CH_3SO_3H$, determined at a concentration of 100%, preferably is from 3 to 20 moles per mole of substrate, particularly from about 4 to 14 moles per mole of substrate.

The hydrogen peroxide preferably is used in amounts in excess of that of the substrate. Generally the amounts range from about 2 to 6 moles $H_2O_2$ per mole of substrate, particularly from about 2.2 to 5 moles per mole of substrate, depending on the COOH groups to be percarboxylated.

The reaction time depends on the nature of the substrate, on the reaction temperature, and the total $H_2SO_4/H_2O$ or $CH_3SO_3H/H_2O$ molar ratio at the end of the reaction. Said ratio preferably ranges from about 1 to 6 and, particularly, from about 1.6 to 4, by choosing suitable values for the various parameters involved.

Reaction times of from about 30 minutes to 2 hours are generally required.

The separation of the imido-aromatic (poly)percarboxylic acid of formula (I) may be carried out according to conventional techniques, such as e.g. filtration of the solid precipitate obtained after treatment of the reaction mixture with an ammonium sulfate solution or solvent extraction.

The imido-aromatic (poly)percarboxylic acids of formula I may thus be obtained in the form of crystalline solids.

The substrates, used as the starting materials, are per se known compounds, or can be prepared according to conventional methods. Examples of suitable substrates are phthalimido-acetic acid, 3-phthalimido-propionic acid, 4-phthalimido-butyric acid, 2-phthalimido-glutaric acid and the corresponding anhydride, 2-phthalimido-succinic acid and the corresponding anhydride, 3-phthalimido-butyric acid, 2-phthalimido-propionic acid, methyl half-ester of 2-phthalimido-glutaric acid, 3-phthalimido-adipic acid, naphthalimido-acetic acid, phthaloyl serine, 4-(4-carboxy)-phthalimido-butyric acid, from which the above preferred peracids of formula (I) are obtained.

The percarboxylic acid products of formula (I) are usually solid at room temperature.

According to the present invention, they may be used in detergent formulations, e.g. granular formulations, as bleaching agents in solution over a wide temperature range, e.g. of from 20 °C to 90 °C.

Therefore, the imido-aromatic peracids of the present invention may be used as bleaching agents either as such, e.g. separate from the detergent composition, or, preferably, associated to and incorporated into conventional detergent compositions which are used within the above defined temperature range and contain other components and/or additives, such as e.g. builders, surfactants, soaps, zeolites, hydrotropic agents, corrosion inhibitors, enzymes, optical brighteners, stabilizers or other brightener compounds.

Preferably, the working temperature ranges from room temperature to about 65 °C.

The preparation and use of the compositions and the corresponding formulations are carried out according to the described and/or conventional techniques.

The imido-aromatic peracids of the present invention may be used in combination with solid and liquid detergent compositions, and/or in the presence of other bleaching (peroxy) compounds.

Further, the present imido-aromatic peracids may be subjected to a known phlegmatization process.

The following examples serve to illustrate the present invention.

The products prepared in the examples were characterized by elemental analysis, determination of their content of active oxygen (by iodometric titration), and by Fourier Transform Infrared Spectroscopy (FT-IR).

Example 1

330 g (3.434 mole) of methanesulphonic acid were placed in a beaker equipped with stirrer, thermometer and external bath.

The internal temperature was increased to 25 °C and 55 g (0.268 mole) of phthalimido-acetic acid were added under stirring within 15 minutes.

The temperature was then lowered to 10 °C and 44 g of $H_2O_2$ (70%, 0.906 mole) were gradually added under stirring, at a rate such as to maintain the temperature below 15 °C.

Stirring was continued at 15 °C for 1.5 hours.

At the end, the reaction mixture was poured into 600 ml of 20% $(NH_4)_2SO_4$, maintained under stirring at 5 °C.

Stirring was continued for 15 minutes at a temperature between 5 °-10 °C.

The solid product was filtered under vacuum over a porous septum. The thus obtained product was suspended in 400 ml of 8% $Na_2SO_4$ and neutralized (pH 6) by addition of a 15% $Na_2CO_3$ solution.

The resulting solid was then filtered, washed with ice water (100 ml), wiped and dried on a porous plate in a $CaCl_2$-drier under vacuum (0.267 kPa = 2 mm Hg) at room temperature.

There were obtained 58 g of substantially pure phthalimido-peracetic acid. Yield: 97%.

The product may be recrystallized by dissolving it in ethyl acetate and by adding petroleum ether until the solution becomes turpid.

Elemental Analysis:

Calculated for $C_{10}H_7O_5N$: C: 54.30%; H: 3.19%; N: 6.33%; O (active): 7.23%.
Found: C: 54.32%; H: 3.33%; N: 6.57%; O (active): 7.2%.
Melting Point: 118 °C (with decomposition).

Example 2

28 g (0.274 mole) of 96% $H_2SO_4$ were introduced into a beaker equipped with stirrer, thermometer and external bath.

The inside temperature was brought to 25 °C and 11.7 g (0.0534 mole) of 3-phthalimido-propionic acid were added under stirring within 15 minutes.

The temperature was lowered to 10 °C and 5.2 g $H_2O_2$ (70%, 0.107 mole) were gradually added under stirring, at a rate such as to maintain the temperature below 15 °C.

The stirring was continued at 15 °C for 1.5 hours. At the end, the reaction mixture was poured into 80 ml of 20% $(NH_4)_2SO_4$, maintained under stirring at 5 °C. Stirring was continued for 15 minutes at a temperature between 5 and 10 °C.

The solid product was filtered under vacuum over a porous septum.

The thus obtained product was suspended in 50 ml of 8% $Na_2SO_4$ and neutralized by adding (pH 6) 15% $Na_2CO_3$. The resulting solid was again filtered, washed with 20 ml of ice water, wiped and dried on a porous plate in a $CaCl_2$-drier under vacuum (0.267 kPa = 2 mm Hg) at room temperature.

11.3 g of substantially pure 3-phthalimido-perpropionic acid were thus obtained. Yield: 90%.

The product may be recrystallized as described in Example 1.

Elemental Analysis:

Calculated for $C_{11}H_9O_5N$: C: 56.17%; H: 3.85%; N: 5.95%; O (active): 6.80%.
Found: C: 56.83%; H: 4.01%; N: 6.10%; O (active): 6.79%.
Melting point: 91 °C (with decomposition).

Example 3

The procedure of Example 2 was repeated, replacing 3-phthalimido-propionic acid by 4-phthalimido-butyric acid (15 g; 0.0643 mole) and using 30 g of 96% $H_2SO_4$ (0.294 mole) and 7 g of $H_2O_2$ (70%, 0.144 mole) and prolonging the reaction time to 2 hours.

14.5 g of substantially pure 4-phthalimido-perbutyric acid were obtained. Yield: 90%.

The product may be recrystallized as described in Example 1.

Elemental Analysis:

Calculated for $C_{12}H_{11}O_5N$: C: 57.83%; H: 4.45%; N: 5.62%; O (active): 6.42%.
Found: C: 57.98%; H: 4.52%; 5.69%; O (active): 6.41%.
Melting point : 103 °C (with decomposition).

Example 4

The procedure of Example 1 was repeated, replacing phthalimido-acetic acid by 2-phthalimido-glutaric acid (6 g; 0.0216 mole) and using 28 g (0.291 mole) of methanesulphonic acid and 3.5 g of $H_2O_2$ (85%, 0.0875 mole).

At the end, 15 ml of 40% $(NH_4)_2SO_4$ were gradually added to the reaction mixture, cooled to 0 °C, at a rate such that the temperature was maintained between 0 and 5 °C.

The resulting mixture was extracted with $Et_2O$ (6 x 30 ml).

The ether extract was washed with 30 ml of 40% $(NH_4)_2SO_4$, dried over anhydrous $Na_2SO_4$, filtered and evaporated.

An oil was obtained which was dissolved in $Et_2O$ (20 ml) and precipitated, as a solid, by adding petroleum ether (40 ml) and maintaining the mixture under agitation up to complete solidification.

After filtration, 5.8 g of 2-phthalimido-diperglutaric acid (95%) were obtained. Yield: 82%.

The product was recrystallized as described in Example 1.

Elemental Analysis:

Calculated for $C_{13}H_{11}O_8N$: C:50.49%; H: 3.58%; N: 4.53%; O (active): 10.34%.
Found: C: 49.96%; H: 3.75%; N:4.70%; O (active): 10.33%.
Melting point : 112 °C (with decomposition).

Example 5

The procedure of Example 4 was repeated, replacing 2-phthalimido-glutaric acid by 2-phthalimido-succinic acid (5 g; 0.019 mole), using 20 g (0.208 mole) of methanesulphonic acid, 3.8 g (0.095 mole) of 85% $H_2O_2$ and extending the reaction time to 2 hours.

At the end, 80 ml of a 40% $(NH_4)_2SO_4$ solution were gradually added to the reaction mixture, cooled to 0 °C, at a rate such that the temperature was maintained between 0 and 5 °C.

Stirring was continued for 15 minutes at 0-5 °C.

The procedure described in Example 2 was then followed.

4 g of substantially pure 2-phthalimido-dipersuccinic acid were obtained. Yield: 71%.

The product may be recrystallized as described in Example 1.

Elemental Analysis:

Calculated for $C_{12}H_9O_8N$: C: 48.82%; H: 3.07%; N: 4.74%; O (active): 10.84%.
Found: C: 48.44%; H: 3.22%; N: 4.88%; O (active) : 10.82%.
Melting point: 131 °C (with decomposition).

Example 6

The procedure of Example 5 was repeated, replacing 2-phthalimido-succinic acid by 2-phthalimido-succinic anhydride (2 g; 0.0082 mole), using 10 g (0.104 mole) of methanesulphonic acid and 1.3 g (0.0325 mole) of 85% $H_2O_2$, and reducing the reaction time to 1.5 hours.

At the end, 60 ml of a 20% $(NH_4)_2SO_4$ solution were gradually added to the reaction mixture, cooled to 0 °C, at a rate such that the temperature was maintained between 0 and 5 °C.

The resulting mixture was extracted with EtOAc/$Et_2O$ 1:2 (2 x 30 ml). The organic extract was washed with 20 ml of a 20% $(NH_4)_2SO_4$ solution, dried over anhydrous $Na_2SO_4$, filtered and evaporated under vacuum.

1.8 g of 2-phthalimido-dipersuccinic acid (95%) were obtained. O (active) found: 10.3%; O (active) calculated for $C_{12}H_9O_8N$: 10.84%.

Example 7

5 g of a 17.4% $Na_2CO_3$ solution were placed in a 50 ml beaker. The internal temperature was brought to 5 °C and 0.8 g of 85% $H_2O_2$ and 0.04 g of $MgSO_4 . 7 H_2O$ were introduced.

While maintaining a temperature of 5 °C, 2 g of 2-phthalimido-succinic anhydride (0.0082 mole) were successively and rapidly added.

The internal temperature was allowed to gradually increase to 20 °C by continuing stirring for 30 minutes.

30 ml of ethyl ether and 4.2 g of 20% $H_2SO_4$ were then added. The ether layer was successively separated, washed with a 40% $(NH_4)_2SO_4$ solution (2 x 20 ml) and dried over anhydrous $Na_2SO_4$. Then, after the filtration of the sulphate, the peracid was precipitated by adding 30 ml of petroleum ether and stirring the mixture at room temperature for 30 minutes. The peracid was filtered and dried under vacuum at room temperature.

1.5 g of 2-phthalimido-mono-persuccinic acid (63%) were obtained.
O (active) found: 3.6%; O (active) calculated for $C_{12}H_9NO_7$ : 5.73%.

Example 8

1.5 g of 85% $H_2O_2$ (0.0375 mole) were added, under stirring at 15-20 °C, to 2 g of a suspension of 4-(4-carboxy)-phthalimido-butyric acid (0.0072 mole) in 12 g (0.125 mole) of methanesulphonic acid.

Stirring was continued for 2 hours at 15 °C.

The reaction product was then poured in 40 ml of a 40% $(NH_4)_2SO_4$ solution maintained at 5 °C and, after 15 minutes of stirring, the separated solid product was filtered. The solid was then neutralized (pH 6) by suspending it in a 8% $Na_2SO_4$ solution and by adding a 15% $Na_2CO_3$ solution.

The resulting solid was again filtered, washed with ice water (30 ml) and dried on a porous plate in a $CaCl_2$ drier.

The product may be recrystallized by dissolving it in ethyl acetate at room temperature and precipitation thereof by adding petroleum ether.

There were thus obtained 2 g of substantially pure 4-(4-percarboxy)-phthalimido-perbutyric acid. Yield: 90%.

Elemental Analysis:

Calculated for $C_{13}H_{11}O_8N$: C: 50.49%; H: 3.58%; N: 4.53%; O (active): 10.35%.
Found: C: 50.04%; H: 3.75%; N: 4.48%; O (active): 10.34%.
Melting point: 109 °C (with decomposition).

Examples 9-12 (Application examples)

Bleaching tests were carried out with the same concentrations of active oxygen in the bleaching solution, and by using the imido-aromatic peracids of the present invention, shown in the following table I, as compared to H.48 peracid (Mg salt of monoperphthalic acid), manufactured by INTEROX Chemical Ltd. London, U.K. for detergent compositions.

All tests were carried out at a constant temperature of 60 °C, with an initial concentration of total active oxygen in the bleach (equal for all products) of 200 mg/l.

Procedure

For each test, 500 ml of deionized water, contained in a 1,000 ml flask equipped with a condenser, were heated to a temperature of 60 °C and adjusted to a pH value of 9.5 (with a few drops of NaOH solution). Then, the bleaching product was added, under stirring, in the amounts given in the following Table 3, and immediately thereafter, two cotton specimens (10 x 10 cm) stained in standard manner by red wine at the EMPA INSTITUTE of St.Gallen (Switzerland), and marked with the "EMPA 114" mark, were added.

The system was subsequently stirred for 60 minutes and, at the end of this time, the specimens, rinsed under running water, were dried and ironed, and were then subjected to the evaluation of the bleaching effect by determining the degree of whiteness by reflectometry. The results are reported in the following Table I, expressed as % Bleaching as defined in the following:

$$\% \text{ Bleaching} = \frac{A - B}{C - B} \times 100$$

wherein:

A = degree of whiteness (%) of the specimen bleached after the test;
B = degree of whiteness (%) of the specimen before the test;
C = degree of whiteness (%) of the completely bleached specimen.

The degree of whiteness was measured by means of an Elrepho Zeiss Reflectometer using a filter N.6 ($\lambda$ = 464 mm) and assuming MgO = 100% of whiteness.

The obtained results show that the peracids of the present invention have a bleaching power which may be compared to that of H.48 and in some cases is even higher.

Table I

| COMPOUND | Amounts used in the test (grams) | Initial concentration of total active oxygen (mg/l) | % Bleaching |
|---|---|---|---|
| - Example 1 (titer: 7.2% of active oxygen) | 1.46 | 200 | 83.6 |
| - Example 2 (titer: 6.79% of active oxygen) | 1.47 | 200 | 83.0 |
| - Example 3 (titer: 6.41% of active oxygen) | 1.56 | 200 | 79.4 |
| - Example 4 (titer: 9.81% of active oxygen) | 1.02 | 200 | 74.0 |
| - Example 5 (titer: 10.82% of active oxygen) | 0.924 | 200 | 75.0 |
| - H 48 (titer: 5.5% of active oxygen) | 1.86 | 200 | 75.1 |

## Claims

**Claims for the following Contracting States : AT, BE, CH, DE, FR, GB, LI, NL, SE**

1. Use of imido-aromatic (poly)percarboxylic acids of general formula (I):

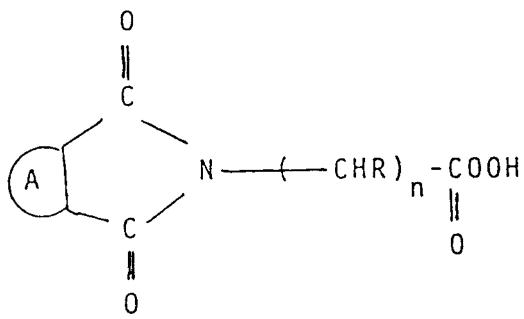

(I)

wherein the groups R, the same or different from each other, represent hydrogen, COOH, COOOH or a linear or branched $C_1$-$C_5$ alkyl group which optionally is substituted with one or more groups X selected from $C_1$-$C_5$ alkoxy, OH, COOH, COOOH, COOR' (R' = $C_1$-$C_5$ alkyl), $NO_2$ and halogen; A represents a benzene or naphthalene ring which optionally is substituted with one or more groups selected from groups R and groups X as defined above; and n is an integer of from 1 to 5; as bleaching agents, either alone or in liquid or solid detergent formulations containing other components and/or additives.

2. Use according to claim 1, wherein said other components and/or additives are selected from builders, surfactants, soaps, zeolites, hydrotropic agents, corrosion inhibitors, enzymes, optical bleaching agents, stabilizers and other peroxy compounds.

3. Imido-aromatic (poly)percarboxylic acids of general formula (I):

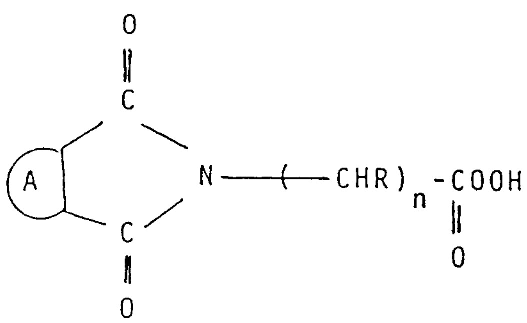

(I)

wherein the groups R, the same or different from each other, represent hydrogen, COOH, COOOH or a linear or branched $C_1$-$C_5$ alkyl group which optionally is substituted with one or more groups X selected from $C_1$-$C_5$ alkoxy, OH, COOH, COOOH, COOR' (R' = $C_1$-$C_5$ alkyl), $NO_2$ and halogen; A represents a benzene or naphthalene ring which optionally is substituted with one or more groups selected from groups R and groups X as defined above; and n is an integer of from 1 to 5; with the exception of L-$\alpha$-phthalimidoperoxypropionic acid, L-$\beta$-phthalimidoperoxybutyric acid and $\alpha$-methyl hydrogen N-phthaloyl-L-peroxyglutamate.

4. (Poly)percarboxylic acids according to claim 3, wherein the group(s) R is (are) selected from straight or branched $C_1$-$C_5$ alkyl groups and hydrogen.

5. Polypercarboxylic acids according to any one of claims 3 and 4, wherein the groups R are hydrogen atoms.

6. (Poly)percarboxylic acids according to any one of claims 3 to 5, wherein said alkyl group(s) R is (are) substituted by one or more, optionally different substituents selected from OH, $NO_2$ and $C_1$-$C_5$ alkoxy.

7. (Poly)percarboxylic acids according to any one of claims 3 to 6, wherein A is an unsubstituted benzene ring.

8. (Poly)percarboxylic acids according to any one of claims 3 to 7, namely phthalimido-peracetic acid, 3-phthalimido-perpropionic acid, 4-phthalimido-perbutyric acid, 2-phthalimido-diperglutaric acid, 2-phthalimido-dipersuccinic acid, 2-phthalimido-monopersuccinic acid, 3-phthalimido-diperadipic acid, naphthalimido-peracetic acid and 4-(4-percarboxy)-phthalimido-perbutyric acid.

9. Process for preparing the (poly)percarboxylic acids according to claim 3, characterized in that a substrate selected from imido-aromatic (poly)carboxylic acids, or anhydrides thereof, corresponding to the desired percarboxylic acid of formula (I), is reacted with concentrated $H_2O_2$ in a medium selected from concentrated $H_2SO_4$ and $CH_3SO_3H$ or in an alkaline medium.

**Claims for the following Contracting State : ES**

1. Use of imido-aromatic (poly)percarboxylic acids of general formula (I):

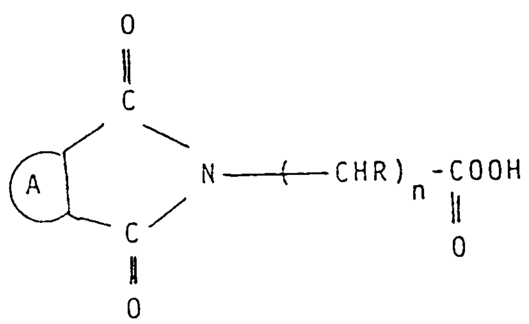

(I)

wherein the groups R, the same or different from each other, represent hydrogen, COOH, COOOH or a linear or branched $C_1$-$C_5$ alkyl group which optionally is substituted with one or more groups X selected from $C_1$-$C_5$ alkoxy, OH, COOH, COOOH, COOR' (R' = $C_1$-$C_5$ alkyl), $NO_2$ and halogen; A represents a benzene or naphthalene ring which optionally is substituted with one or more groups selected from groups R and groups X as defined above; and n is an integer of from 1 to 5; as bleaching agents, either alone or in liquid or solid detergent formulations containing other components and/or additives.

2. Use according to claim 1, wherein said other components and/or additives are selected from builders, surfactants, soaps, zeolites, hydrotropic agents, corrosion inhibitors, enzymes, optical bleaching agents, stabilizers and other peroxy compounds.

3. Imido-aromatic (poly)percarboxylic acids of general formula (I):

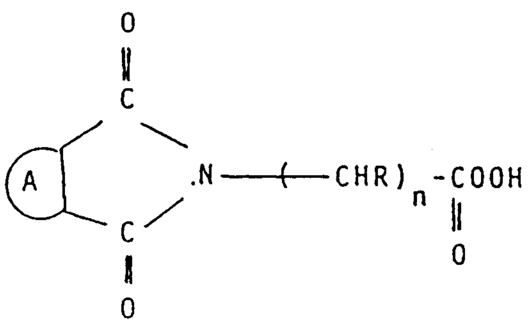

(I)

wherein the groups R, the same or different from each other, represent hydrogen, COOH, COOOH or a linear or branched $C_1$-$C_5$ alkyl group which optionally is substituted with one or more groups X selected from $C_1$-$C_5$ alkoxy, OH, COOH, COOOH, COOR' (R' = $C_1$-$C_5$ alkyl), $NO_2$ and halogen; A represents a

benzene or naphthalene ring which optionally is substituted with one or more groups selected from groups R and groups X as defined above; and n is an integer of from 1 to 5; with the exception of L-α-phthalimidoperoxypropionic acid, L-β-phthalimidoperoxybutyric acid and α-methyl hydrogen N-phthaloyl-L-peroxyglutamate.

**4.** (Poly)percarboxylic acids according to claim 3, wherein the group(s) R is (are) selected from straight or branched $C_1$-$C_5$ alkyl groups and hydrogen.

**5.** Polypercarboxylic acids according to any one of claims 3 and 4, wherein the groups R are hydrogen atoms.

**6.** (Poly)percarboxylic acids according to any one of claims 3 to 5, wherein said alkyl group(s) R is (are) substituted by one or more, optionally different substituents selected from OH, $NO_2$ and $C_1$-$C_5$ alkoxy.

**7.** (Poly)percarboxylic acids according to any one of claims 3 to 6, wherein A is an unsubstituted benzene ring.

**8.** (Poly)percarboxylic acids according to any one of claims 3 to 7, namely phthalimido-peracetic acid, 3-phthalimido-perpropionic acid, 4-phthalimido-perbutyric acid, 2-phthalimido-diperglutaric acid, 2-phthalimido-dipersuccinic acid, 2-phthalimido-monopersuccinic acid, 3-phthalimido-diperadipic acid, naphthalimido-peracetic acid and 4-(4-percarboxy)-phthalimido-perbutyric acid.

**9.** Process for preparing the (poly)percarboxylic acids of general formula (I):

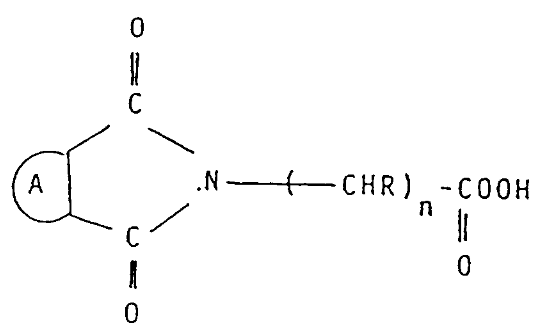

(I)

wherein the groups R, the same or different from each other, represent hydrogen, COOH, COOOH or a linear or branched $C_1$-$C_5$ alkyl group which optionally is substituted with one or more groups X selected from $C_1$-$C_5$ alkoxy, OH, COOH, COOOH, COOR' (R' = $C_1$-$C_5$ alkyl), $NO_2$ and halogen; A represents a benzene or naphthalene ring which optionally is substituted with one or more groups selected from groups R and groups X as defined above; and n is an integer of from 1 to 5; with the exception of L-α-phthalimidoperoxypropionic acid, L-β-phthalimidoperoxybutyric acid and α-methyl hydrogen N-phthaloyl-L-peroxyglutamate, characterized in that a substrate selected from imido-aromatic (poly)-carboxylic acids, or anhydrides thereof, corresponding to the desired percarboxylic acid of formula (I), is reacted with concentrated $H_2O_2$ in a medium selected from concentrated $H_2SO_4$ and $CH_3SO_3H$ or in an alkaline medium.

**Patentansprüche**
**Patentansprüche für folgende Vertragsstaaten : AT, BE, CH, DE, FR, GB, LI, NL, SE**

1. Verwendung imidoaromatischer (Poly)percarbonsäuren der allgemeinen Formel (I):

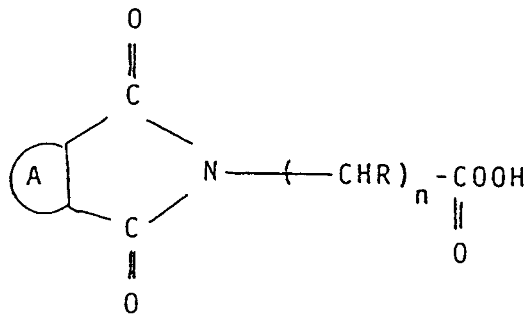

(I)

worin die Gruppen R, die gleich oder voneinander verschieden sein können, Wasserstroff, COOH, COOOH oder eine lineare oder verzweigte $C_1$-$C_5$-Alkylgruppe bedeuten, die fakultativ mit einer oder mehreren Gruppen X, ausgewählt aus $C_1$-$C_5$-Alkoxy, OH, COOH, COOOH, COOR' (R' = $C_1$-$C_5$-Alkyl), $NO_2$ und Halogen, substituiert sein kann; A einen Benzol- oder Naphthalinring bedeutet, der fakultativ mit einer oder mehreren Gruppen, ausgewählt aus den oben definierten Gruppen R und Gruppen X, substituiert ist, und n eine ganze Zahl von 1 bis 5 ist, als Bleichmittel, entweder allein oder in flüssigen oder festen Reinigungsmittelformulierungen, die andere Komponenten und/oder Zusätze enthalten.

2. Verwendung nach Anspruch 1, worin die anderen Komponenten und/oder Zusätze ausgewählt sind aus Waschhilfsmitteln, oberflächenaktiven Mitteln, Seilen, Zeolithen, hydrotropen Mitteln, Korrosionsinhibitoren, Enzymen, optischen Bleichmitteln, Stabilisatoren und anderen Peroxyverbindungen.

3. Imidoaromatische (Poly)percarbonsäuren der allgemeinen Formel (I):

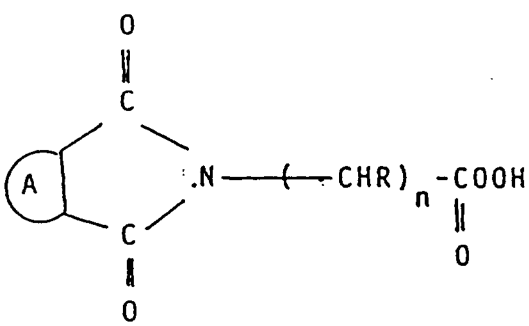

(I)

worin die Gruppen R, die gleich oder voneinander verschieden sein können, Wasserstroff, COOH, COOOH oder eine lineare oder verzweigte $C_1$-$C_5$-Alkylgruppe bedeuten, die fakultativ mit einer oder mehreren Gruppen X, ausgewählt aus $C_1$-$C_5$-Alkoxy, OH, COOH, COOOH, COOR', (R' = $C_1$-$C_5$-Alkyl), $NO_2$ und Halogen, substituiert sein kann; A einen Benzol- oder Naphthalinring bedeutet, der fakultativ mit einer oder mehreren Gruppen, ausgewählt aus den oben definierten Gruppen R und Gruppen X, substituiert ist, und n eine ganze Zahl von 1 bis 5 ist, mit Ausnahme von L-alpha-Phthalimidoperoxypropionsäure, L-beta-Phthalimidoperoxybuttersäure und alpha-Methyl-H-N-phthaloyl-L-peroxyglutamat.

4. (Poly)percarbonsäuren nach Anspruch 3, worin die Gruppe(n) R aus geraden oder verzweigten $C_1$-$C_5$-Alkylgruppen und Wasserstoff ausgewählt ist (sind).

5. (Poly)percarbonsäuren nach irgendeinem der Ansprüche 3 und 4, worin die Gruppen R Wasserstoffatome sind.

6. (Poly)percarbonsäuren nach irgendeinem der Ansprüche 3 bis 5, worin die Alkylgruppe(n) R durch einen oder mehrere fakultativ verschiedene Substituenten, ausgewählt aus OH, $NO_2$ und $C_1$-$C_5$-Alkoxy, substituiert ist bzw. sind.

7. (Poly)percarbonsäuren nach irgendeinen der Ansprüche 3 bis 6, worin A ein unsubstituierter Benzolring ist.

8. (Poly)percarbonsäuren nach irgendeinem der Ansprühe 3 bis 7, nämlich Phthalimidoperessigsäure, 3-Phthalimidoperpropionsäure, 4-Phthalimidoperbuttersäure, 2-Phthalimidodiperglutarsäure, 2-Phthalimidodiperbernsteinsäure, 2-Phthalimidomonoperbernsteinsäure, 3-Phthalimidodiperadipinsäure, Naphthalimidoperessigsäure und 4-(4-Percarboxy)-phthalimidoperbuttersäure.

9. Verfahren zur Herstellung der (Poly)percarbonsäuren nach Anspruch 3, dadurch gekennzeichnet, daß ein Substrat, ausgewählt aus imidoaromatischen (Poly)carbonsäuren oder deren Anhydriden entsprechend der gewünschten Percarbonsäure der Formel (I), mit konzentriertem $H_2O_2$ in einem Medium, ausgewählt aus $H_2SO_4$ und $CH_3SO_3H$, oder in einem alkalischen Medium umgesetzt wird.

**Patentansprüche für folgenden Vertragsstaat : ES**

1. Verwendung imidoaromatischer (Poly)percarbonsäuren der allgemeinen Formel (I):

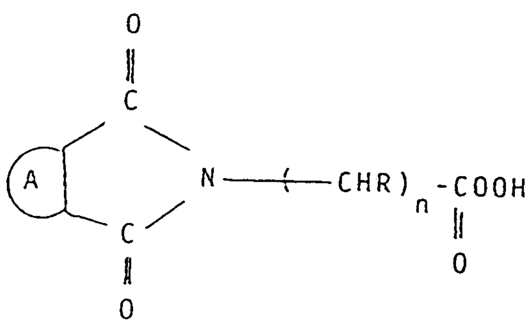

(I)

worin die Gruppen R, die gleich oder voneinander verschieden sein können, Wasserstroff, COOH, COOOH oder eine lineare oder verzweigte $C_1$-$C_5$-Alkylgruppe bedeuten, die fakultativ mit einer oder mehreren Gruppen X, ausgewählt aus $C_1$-$C_5$-Alkoxy, OH, COOH, COOOH, COOR' (R' = $C_1$-$C_5$-Alkyl), $NO_2$ und Halogen, substituiert sein kann; A einen Benzol- oder Naphthalinring bedeutet, der fakultativ mit einer oder mehreren Gruppen, ausgewählt aus den oben definierten Gruppen R und Gruppen X, substituiert ist, und n eine ganze Zahl von 1 bis 5 ist, als Bleichmittel, entweder allein oder in flüssigen oder festen Reinigungsmittelformulierungen, die andere Komponenten und/oder Zusätze enthalten.

2. Verwendung nach Anspruch 1, worin die anderen Komponenten und/oder Zusätze ausgewählt sind aus Waschhilfsmitteln, oberflächenaktiven Mitteln, Seifen, Zeolithen, hydrotropen Mitteln, Korrosionsinhibitoren, Enzymen, optischen Bleichmitteln, Stabilisatoren und anderen Peroxyverbindungen.

3. Imidoaromatische (Poly)percarbonsäuren der allgemeinen Formel (I):

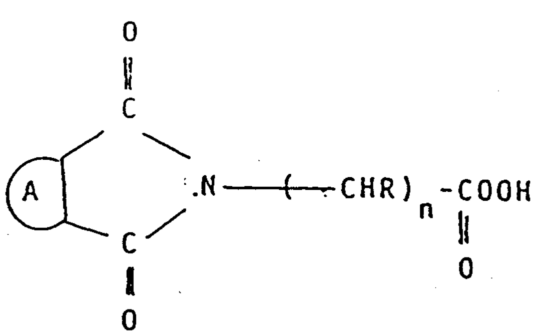

(I)

worin die Gruppen R, die gleich oder voneinander verschieden sein können, Wasserstroff, COOH, COOOH oder eine lineare oder verzweigte $C_1$-$C_5$-Alkylgruppe bedeuten, die fakultativ mit einer oder mehreren Gruppen X, ausgewählt aus $C_1$-$C_5$-Alkoxy, OH, COOH, COOOH, COOR', (R' = $C_1$-$C_5$-Alkyl), $NO_2$ und Halogen, substituiert sein kann; A einen Benzol- oder Naphthalinring bedeutet, der fakultativ mit einer oder mehreren Gruppen, ausgewählt aus den oben definierten Gruppen R und Gruppen X, substituiert ist, und n eine ganze Zahl von 1 bis 5 ist, mit Ausnahme von L-alpha-Phthalimidoperoxypropionsäure, L-beta-Phthalimidoperoxybuttersäure und alpha-Methyl-H-N-phthaloyl-L-peroxyglutamat.

4. (Poly)percarbonsäuren nach Anspruch 3, worin die Gruppe(n) R aus geraden oder verzweigten $C_1$-$C_5$-Alkylgruppen und Wasserstoff ausgewählt ist (sind).

5. (Poly)percarbonsäuren nach irgendeinem der Ansprüche 3 und 4, worin die Gruppen R Wasserstoffatome sind.

6. (Poly)percarbonsäuren nach irgendeinem der Ansprüche 3 bis 5, worin die Alkylgruppe(n) R durch einen oder mehrere fakultativ verschiedene Substituenten, ausgewählt aus OH, $NO_2$ und $C_1$-$C_5$-Alkoxy, substituiert ist bzw. sind.

7. (Poly)percarbonsäuren nach irgendeinen der Ansprüche 3 bis 6, worin A ein unsubstituierter Benzolring ist.

8. (Poly)percarbonsäuren nach irgendeinem der Ansprüche 3 bis 7, nämlich Phthalimidoperessigsäure, 3-Phthalimidoperpropionsäure, 4-Phthalimidoperbuttersäure, 2-Phthalimidodiperglutarsäure, 2-Phthalimidodiperbernsteinsäure, 2-Phthalimidomonoperbernsteinsäure, 3-Phthalimidodiperadipinsäure, Naphthalimidoperessigsäure und 4-(4-Percarboxy)-phthalimidoperbuttersäure.

9. Verfahren zur Herstellung der (Poly)percarbonsäuren der allgemeinen Formel (I):

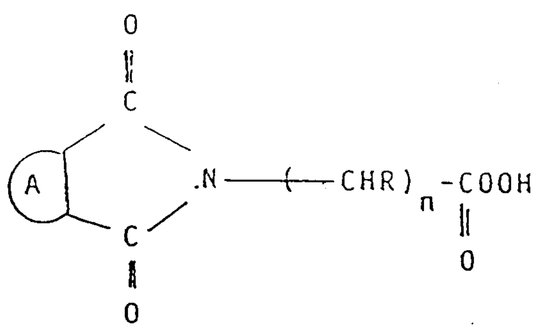

(I)

worin die Gruppen R, die gleich oder voneinander verschieden sein können, Wasserstroff, COOH, COOOH oder eine lineare oder verzweigte $C_1$-$C_5$-Alkylgruppe bedeuten, die fakultativ mit einer oder mehreren Gruppen X, ausgewählt aus $C_1$-$C_5$-Alkoxy, OH, COOH, COOOH, COOR', (R' = $C_1$-$C_5$-Alkyl), $NO_2$ und Halogen, substituiert sein kann; A einen Benzol- oder Naphthalinring bedeutet, der fakultativ mit einer oder mehreren Gruppen, ausgewählt aus den oben definierten Gruppen R und Gruppen X, substituiert ist, und n eine ganze Zahl von 1 bis 5 ist, mit Ausnahme von L-alpha-Phthalimidoperoxypropionsäure, L-beta-Phthalimidoperoxybutterzäure und alpha-Methyl-H-N-phthaloyl-L-peroxyglutamat,
dadurch gekennzeichnet, daß ein Substrat, ausgewählt aus imidoaromatischen (Poly)carbonsäuren oder deren Anhydriden entsprechend der gewünschten Percarbonsäure der Formel (I), mit konzentriertem $H_2O_2$ in einem Medium, ausgewählt aus $H_2SO_4$ und $CH_3SO_3H$, oder in einem alkalischen Medium umgesetzt wird.

**Revendications**

**Revendications pour les Etats contractants suivants : AT, BE, CH, DE, FR, GB, LI, NL, SE**

1. Utilisation d'acides imido-aromatiques (poly)percarboxyliques de formule générale (I):

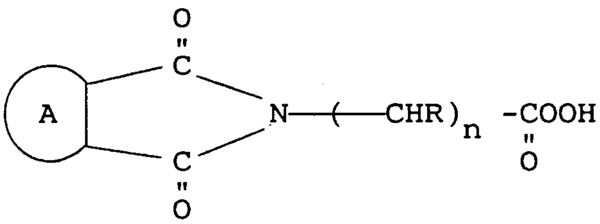

(I)

dans laquelle:
les groupes
- R, identiques ou différents les uns des autres, représentent un atome d'hydrogène, COOH, COOOH ou un groupe alkyle en $C_1$ à $C_5$, linéaire ou ramifié, qui est éventuellement substitué par un ou plusieurs groupes X sélectionnés parmi les groupes alcoxy en $C_1$ à $C_5$, OH, COOH, COOOH, COOR' (R' = un groupe alkyle en $C_1$ à $C_5$), $NO_2$ et un atome d'halogène;
- A représente un cycle benzène ou naphtalène qui est éventuellement substitué par un ou plusieurs groupes sélectionnés parmi les groupes R et les groupes X tels que définis ci-dessus; et
- n est un nombre entier de 1 à 5;

comme agents de blanchiment, soit seuls, soit contenus dans des formulations détergentes solides ou liquides contenant d'autres composants et/ou additifs.

2. Utilisation selon la revendication 1, dans laquelle lesdits autres composants et/ou additifs sont sélectionnés parmi des adjuvants tensioactifs, savons, zéolites, agents hydrotropes, inhibiteurs de corrosion, enzymes, agents brillanteurs optiques, stabilisateurs et autres composés peroxy.

3. Acides imido-aromatiques (poly)peroxycarboxyliques de formule générale (I):

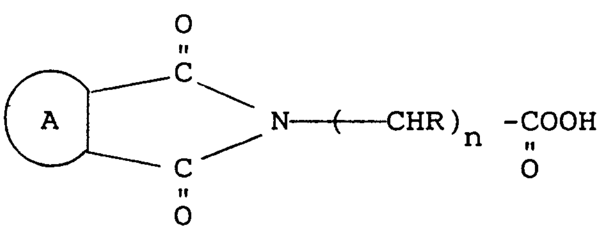

(I)

dans laquelle:
les groupes
- R, identiques ou différents les uns des autres, représentent un atome d'hydrogène, COOH, COOOH ou un groupe alkyle en $C_1$ à $C_5$, linéaire ou ramifié, qui est éventuellement substitué par un ou plusieurs groupes X sélectionnés parmi les groupes alcoxy en $C_1$ à $C_5$, OH, COOH, COOOH, COOR' (R' = un groupe alkyle en $C_1$ à $C_5$), $NO_2$ et un atome d'halogène;
- A représente un cycle benzène ou naphtalène qui est éventuellement substitué par un ou plusieurs groupes sélectionnés parmi les groupes R et les groupes X tels que définis ci-dessus; et
- n est un nombre entier de 1 à 5;

avec l'exception de l'acide L-$\alpha$-phtalimidoperoxypropionique, l'acide L-$\beta$-phtalimidoperoxybutyrique et l'$\alpha$-méthylhydrogéno-N-phtaloyl-L-peroxyglutamate.

4. Les acides (poly)percarboxyliques selon la revendication 3, dans lesquels les groupes R est ( ou sont) sélectionné(s) parmi les groupes alkyle en $C_1$ à $C_5$ à chaîne droite ou ramifiée, et l'atome d'hydrogène.

5. Acides (poly)percarboxyliques selon les revendications 3 ou 4, dans lesquels les groupes R sont des atomes d'hydrogène.

6. Acides (poly)percarboxyliques selon l'une quelconque des revendications 3 à 5, dans lesquels le(s)dit-(s) groupe(s) alkyle R est (ou sont) substitué(s) par un ou plusieurs, éventuellement différents substituants, sélectionnés parmi OH, $NO_2$ et des groupes alcoxy en $C_1$ à $C_5$.

7. Acides (poly)percarboxyliques selon l'une quelconque des revendications 3 à 6, dans lesquels A est un cycle benzène non-substitué.

8. Acides (poly)percarboxyliques selon l'une quelconque des revendications 3 à 7, nommément acide phtalimidoperacétique, acide 3-phtalimidoperpropionique, acide 4-phtalimidoperbutyrique, acide 2-phtalimidodiperglutarique, acide 2-phtalimidodipersuccinique, acide 2-phtalimidomonopersuccinique, acide 3-phtalimidoperadipique, acide naphtalimidoperacétique et acide 4-(4-percarboxy)-phtalimidoperbutyrique.

9. Procédé pour la préparation d'acides (poly)percarboxyliques selon la revendication 3, caractérisé en ce que le substrat sélectionné parmi les acides imido-aromatiques (poly)carboxyliques ou leurs anhydrides correspondant aux acides percarboxyliques de formule (I) désirés réagit avec $H_2O_2$ concentré dans un milieu sélectionné parmi $H_2SO_4$ concentré et $CH_3SO_3H$ concentré ou un milieu alcalin.

**Revendications pour l'Etat contractant suivant : ES**

1. Utilisation d'acides imido-aromatiques (poly)percarboxyliques de formule générale (I):

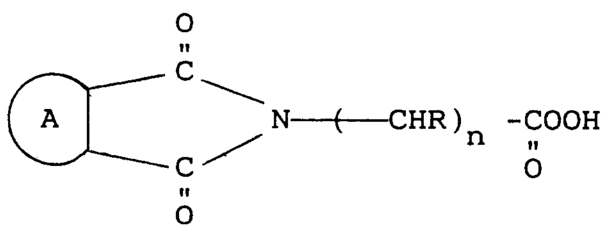

(I)

dans laquelle:
les groupes
- R, identiques ou différents les uns des autres, représentent un atome d'hydrogène, COOH, COOOH ou un groupe alkyle en $C_1$ à $C_5$, linéaire ou ramifié, qui est éventuellement substitué par un ou plusieurs groupes X sélectionnés parmi les groupes alcoxy en $C_1$ à $C_5$, OH, COOH, COOOH, COOR' (R' = un groupe alkyle en $C_1$ à $C_5$), $NO_2$ et un atome d'halogène;
- A représente un cycle benzène ou naphtalène qui est éventuellement substitué par un ou plusieurs groupes sélectionnés parmi les groupes R et les groupes X tels que définis ci-dessus; et
- n est un nombre entier de 1 à 5;

comme agents de blanchiment, soit seuls, soit contenus dans des formulations détergentes solides ou liquides contenant d'autres composants et/ou additifs.

2. Utilisation selon la revendication 1, dans laquelle lesdits autres composants et/ou additifs sont sélectionnés parmi des adjuvants, tensioactifs, savons, zéolites, agents hydrotropes, inhibiteurs de corrosion, enzymes, agents brillanteurs optiques, stabilisateurs et autres composés peroxy.

**3.** Acides imido-aromatiques (poly)peroxycarboxyliques de formule générale (I):

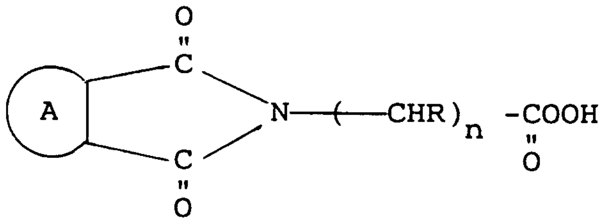

(I)

dans laquelle:
les groupes

- R, identiques ou différents les uns des autres, représentent un atome d'hydrogène, COOH, COOOH ou un groupe alkyle en $C_1$ à $C_5$, linéaire ou ramifié, qui est éventuellement substitué par un ou plusieurs groupes X sélectionnés parmi les groupes alcoxy en $C_1$ à $C_5$, OH, COOH, COOOH, COOR' (R' = un groupe alkyle en $C_1$ à $C_5$), $NO_2$ et un atome d'halogène;
- A représente un cycle benzène ou naphtalène qui est éventuellement substitué par un ou plusieurs groupes sélectionnés parmi les groupes R et les groupes X tels que définis ci-dessus; et
- n est un nombre entier de 1 à 5;

avec l'exception de l'acide L-$\alpha$-phtalimidoperoxypropionique, l'acide L-$\beta$-phtalimidoperoxybutyrique et l'$\alpha$-méthylhydrogéno-N-phtaloyl-L-peroxyglutamate.

**4.** Les acides (poly)percarboxyliques selon la revendication 3, dans lesquels le(s) groupe(s) R est (ou sont) sélectionné(s) parmi les groupes alkyle en $C_1$ à $C_5$ à chaîne droite ou ramifiée, et l'atome d'hydrogène.

**5.** Acides (poly)percarboxyliques selon les revendications 3 ou 4, dans lesquels les groupes R sont des atomes d'hydrogène.

**6.** Acides (poly)percarboxyliques selon l'une quelconque des revendications 3 à 5, dans lesquels le(s)dit-(s) groupe(s) alkyle R est (ou sont) substitué(s) par un ou plusieurs, éventuellement différents substituants, sélectionnés parmi OH, $NO_2$ et des groupes alcoxy en $C_1$ à $C_5$.

**7.** Acides (poly)percarboxyliques selon l'une quelconque des revendications 3 à 6, dans lesquels A est un cycle benzène non-substitué.

**8.** Acides (poly)percarboxyliques selon l'une quelconque des revendications 3 à 7, nommément acide phtalimidoperacétique, acide 3-phtalimidoperpropionique, acide 4-phtalimidoperbutyrique, acide 2-phtalimidodiperglutarique, acide 2-phtalimidodipersuccinique, acide 2-phtalimidomonopersuccinique, acide 3-phtalimidoperadipique, acide naphtalimidoperacétique et acide 4-(4-percarboxy)-phtalimidoperbutyrique.

**9.** Procédé pour la préparation d'acides (poly)percarboxyliques de formule générale (I):

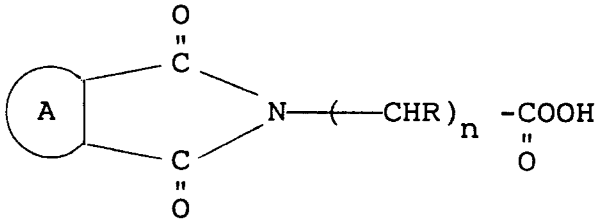

(I)

dans laquelle:

les groupes

- R, identiques ou différents les uns des autres, représentent un atome d'hydrogène, COOH, COOOH ou un groupe alkyle en $C_1$ à $C_5$, linéaire ou ramifié, qui est éventuellement substitué par un ou plusieurs groupes X sélectionnés parmi les groupes alcoxy en $C_1$ à $C_5$, OH, COOH, COOOH, COOR' (R' = un groupe alkyle en $C_1$ à $C_5$), $NO_2$ et un atome d'halogène;
- A représente un cycle benzène ou naphtalène qui est éventuellement substitué par un ou plusieurs groupes sélectionnés parmi les groupes R et les groupes X tels que définis ci-dessus; et
- n est un nombre entier de 1 à 5;

avec l'exception de l'acide L-α-phtalimidoperoxypropionique, l'acide L-β-phtalimidoperoxybutyrique et l'α-méthylhydrogéno-N-phtaloyl-L-peroxyglutamate,
caractérisé en ce que le substrat sélectionné parmi les acides imido-aromatiques (poly)carboxyliques ou leurs anhydrides correspondant aux acides percarboxyliques de formule (I) désirés réagit avec $H_2O_2$ concentré dans un milieu sélectionné parmi $H_2SO_4$ concentré et $CH_3SO_3H$ concentré ou un milieu alcalin.